# EUROPEAN PATENT APPLICATION

(11) **EP 3 915 483 A1**
(43) Date of publication of application: **01.12.2021**
(21) Application number: 21184197.8
(22) Date of filing: 28.09.2015
(51) Int. Cl.: A61B 6/14, A61B 6/00, A61B 6/04, A61B 6/06

(54) **APPARATUS FOR ACQUIRING PANORAMIC, OPTIONALLY 3D VOLUMETRIC CBCT, OPTIONALLY TELERADIOGRAPHIC IMAGES**

(30) Priority: 29.09.2014 IT BO20140532; 29.09.2014 IT BO20140533; 29.09.2014 IT BO20140534
(62) Divisional of application: 15187166.2
(71) Applicant: CEFLA SOCIETA' COOPERATIVA, 40026 Imola (BO) (IT)
(72) Inventor: RIGHINI, Dario, 40026 Imola (IT); BENEDETTI, Jarno, 48010 Fusignano (IT); BALDINI, Antonio, 40026 Imola (IT); SALSINI, Sergio, 40013 Castel Maggiore (IT)
(74) Representative: Karaghiosoff, Giorgio Alessandro

(57) **Abstract**

An apparatus (11, 21, 31) for acquiring panoramic and optionally CBCT volumetric images, comprising a X-ray source (5) projecting a collimated X-ray bundle through a patient, a bi-dimensional X-ray sensor (6) placed so as to measure the intensity of the radiation after it crossed the patient, a support group supporting said source (5) and sensor (6), which group comprises a C-arm (4) to which said sensor and said source are fixed/fixable, said C arm (4) being fixed to a vertically mobile support (3) supported by a post (2); a mechanical system capable to allow the rotation and the translation of said C-arm around the patient, so as to acquire radiographic images from different positions; a patient positioning device (7) for positioning the patient in an intermediate position between said X-ray source (5) and said sensor (6);

According to the invention, the said mobile support (3) having a C shape and wherein said mobile support (3) and said support group (400 5, 6) have a double C configuration and wherein the C arm of said mobile support (3) and of the said support group (400, 5, 6) are opposed to each other with their top arms or ends overlapping to each other amd furthermore wherein said C or set down C shaped arm of the said mobile support (400) comprises a second lower arm (402), parallel to a first arm (401) articulated to a mobile portion (62), supporting at its free end said X-ray sensor (6), said X-ray source (5) being fixed at the lower end of a third transversal arm (403) linking said horizontal or substantially horizontal arms (401) and (402), while the position of the free end of arm (402) to which sensor (6) is fixed is such that sensor and source are diametrically opposed to each other with respect to the position of the patient as requested for acquiring radiographic images.

## Description

The present invention relates to the technical field of the extraoral dental radiography, and in particular to an apparatus capable of performing panoramic, optionally CBCT 3D volumetric, optionally teleradiographic radiographies. All these kinds of radiographies are well known in the art.

In particular, the present invention relates to a radiographic apparatus capable of acquiring the said kinds of acquisitions, whose C-arm can have two positions, a use position and a rest position; the rest position is the position having the minimal dimensions.

Panoramic radiography (also known as orthopantomography) produces a radiographic image of a curved plan approximating patient jaws, with blurring of the anatomical structures laying outside a narrow layer around the predesigned curved plane. This kind of acquisition is also indicated in the following with PAN.

Cone beam volumetric radiography (also known as CBCT) is the acquisition, from different projection angles, of a series of two-dimensional radiographic images which will be processed post-acquisition to reconstruct three-dimensional volumes.

Teleradiography is a projective radiographic technique, producing radiographic images of the skull or of other anatomical areas from different projections, with minimum magnification and geometrical distortion. Usually two perspectives are represented, latero-lateral and anteroposterior. In the following, teleradiography will be abbreviated as CEPH.

The panoramic technique has been known since the '50s, and is normally performed with apparatuses having a consolidated structure, allowing to rotate the X-ray source and sensor around the patient during the acquisition of the image. Such apparatuses typically comprise a base from which a post rises, supporting a mechanism vertically shifting a C-arm, which on one end carries a X-ray source and a primary collimator immediately downstream the X-ray exit, and on the other end a X-ray sensor. Between source and sensor a device is placed for positioning the patient, keeping her/him in a predefined position.

Teleradiography requires to have a X-ray source-sensor distance markedly higher than that required for panoramic and CBCT volumetric acquisitions (indicatively 450-700 mm for PAN e CBCT acquisitions, 1400-1800 mm for CEPH acquisitions). These different distances oblige the manufacturers to provide an arm carrying a suitable CEPH sensor in order to allow the dentist to perform the different kinds of acquisitions according to her/his needs.

Morita US4365340 patent, filed in 1980, is just an example of a long series of patents describing apparatuses having the above-described structure. Typically such apparatuses have a structure where the post supporting the C-arm rests on the floor, with a base which generally is of notable dimensions.

Such apparatuses allow to move the C-arm, supporting on one end the X-ray source, and on the other end the X-ray sensor, on a plan parallel to the floor. Traditionally, X indicates the movement along the right-left axis of the patient, while Y indicates the movement along the sagittal axis of the patient. The movement in this plane can be obtained through three motors (X, Y, R), or through two motors only (Y and R). The solution using two motors, Y and R, is cheaper and more compact; nonetheless, it allows to perform practically all the necessary trajectories.

The apparatuses of this kind have the disadvantage of being quite cumbersome. Moreover, their use in the dental practice is intermittent; many days may go by without the need to perform a panoramic, a CBCT volumetric or a teleradiographic acquisition. As a consequence, the reduced dimensions of the apparatus is a feature particularly appreciated.

Moreover, the presence of the floor base can pose some safety risks, in that the base must be sufficiently broad to give stability to the apparatus, and as a consequence it exposes patient and operators to the risk of stumbling.

A way to reduce the dimensions of the panoramic apparatuses having the traditional structure, and to increase their safety, is to fix the apparatus to a wall instead of supporting it through a floor base. A wall panoramic apparatus is described in Morita patent US4002915 filed in 1974.

Another panoramic wall apparatus is described in Vatech application KR20100043334A filed in 2008. This application describes an apparatus fixed to the ceiling or to a wall, capable of acquiring PAN, CBCT and CEPH acquisitions.

Document US7486767 discloses an X-ray apparatus according to the preamble of claim 1. The vertically mobile support for the x-ray imaging group does not show a telescopic post. The device disclosed is in the stand alone configuration and does not need any attachment to a wall or similar stationary structure.

Document US6118842 discloses a dental X-ray apparatus having a post on which a vertically mobile support slides, the said support carrying the X-ray imaging group consisting in the C-arm bearing the X-ray source and the X-ray sensor at its opposite ends.

Also in this case a stand alone configuration is shown and no arrangement is provided for allowing attachment of the post to a wall. Furthermore, no telescopic post is provided.

Both documents show a configuration in which the C-arm bearing the source and the sensor and of the C-arm of the vertically mobile support are not oriented in a counter posed position one to the other, i.e. with the opening of the C configurations of the x ray imaging group and of the vertically mobile support facing each other. The vertically slidable support has a C form oriented with the opening on a vertical side, while the C-arm of the x ray imaging group is oriented downwards. The C-arm of the X-ray imaging group is attached to an horizontally cantilevered arm of the vertically mobile support with its intermediate segment linking the two parallel and downwards oriented arms carrying respectively the X-ray source and the X-ray sensor.
Furthermore, the horizontally cantilevered arm of the vertically mobile support has a fixed length.

Aim of the present invention is providing an apparatus having reduced dimensions capable of performing, after a suitable set-up, the three kinds of radiographic acquisitions.

A further aim of the present invention is providing an apparatus which is particularly simple and cheap to produce.

This aim is obtained with an apparatus having the features of the independent claim. Advantageous embodiments and refinements are specified in claims dependent thereon.

In particular the apparatus has the support of the C-arm, supporting on one end the X-ray source and on the other end the sensor, fixed to a wall. This allows to markedly reduce the dimensions of the overall apparatus while not in use.

Further advantages and properties of the present invention are disclosed in the following description, in which exemplary embodiments of the present invention are explained in detail on the basis of the drawings, showing:
Figure 1 Wall apparatus, side view;
Figure 2 Floor-fixed, wall-mounted set-up apparatus, side view;
Figure 3 Stand alone apparatus, side view;
Figure 4 Detail of the internal post, axonometric view
Figure 5a Section of the external post in one of its two extreme positions;
Figure 5b Section of the external post in the other of its two extreme positions;
Figure 6 Apparatus with telescopic strut, side view;
Figure 7 Detail of the apparatus with telescopic strut, axonometric view;
Figure 8 Schematic representation of the new configuration compared to the prior art;
Figure 9 Detail of the chin support, axonometric view;
Figure 10 Detail of the storage compartment.

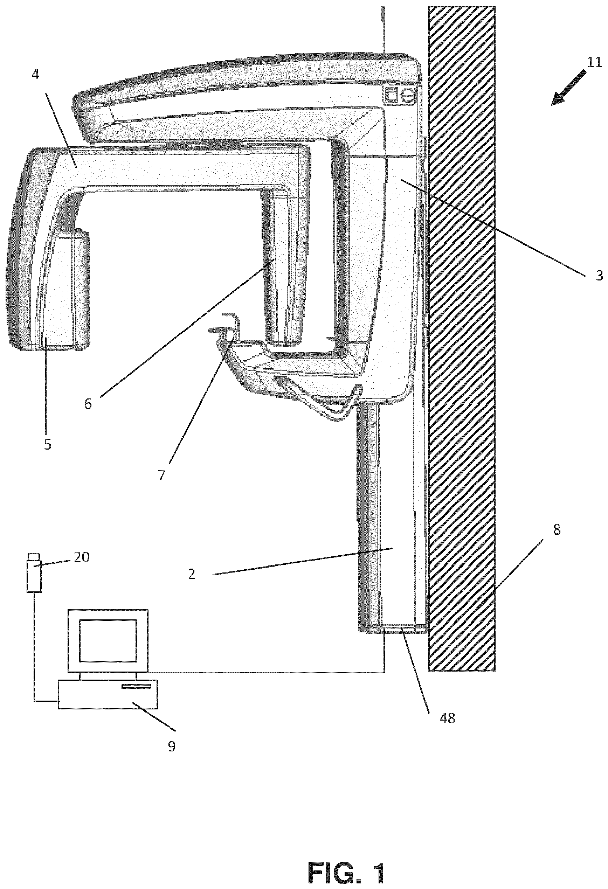

In Figure 1, 11 indicates a wall apparatus capable of performing panoramic radiographies. The apparatus 11 comprises a post 2 supporting a support 3. Support 3 supports a C-arm 4 which is fixed to the horizontal section of support 3. The C-arm 4 in its turn supports at one of its ends an X-ray source 5, and at the opposed end an X-ray sensor 6. The support 3 is vertically movable in order to adjust it to the different heights of individual patients. Moreover, a device 7 for the positioning of the patient is present, which is also fixed to support 3.

The apparatus 11 is entirely supported by a wall 8 to which it is fixed.

In an embodiment, the back of fixed post 41 can be directly fixed to the wall through suitable plugs.

In an alternative embodiment a metallic counter-plate is present (not shown), useful in the case of a structurally insufficient wall. The metallic counter-plate must be bricked in the wall supporting the apparatus 11.

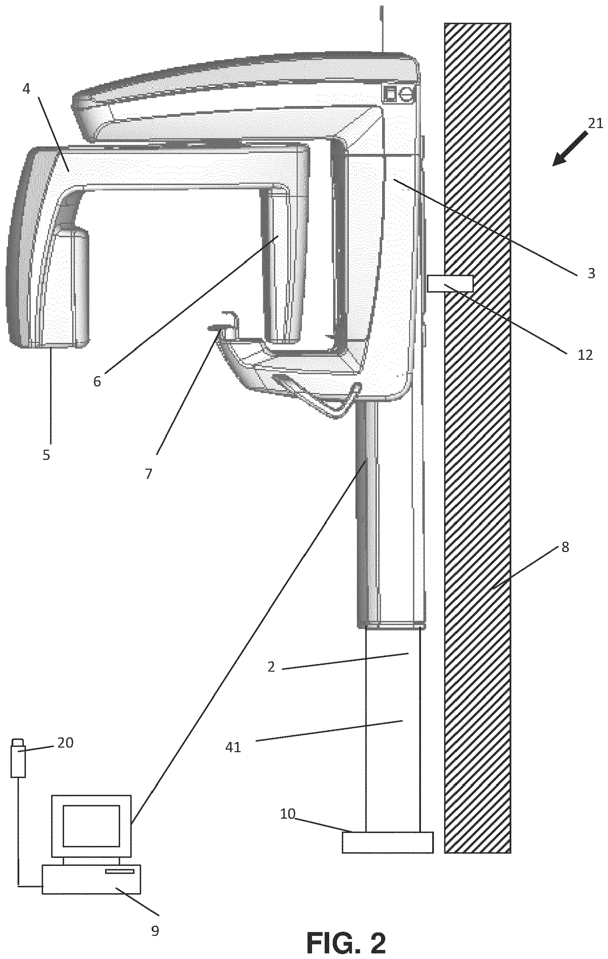

In Figure 2, 21 indicates a floor-fixed, wall-mounted set-up apparatus capable of performing panoramic radiographies. The apparatus 21 comprises a post 2 supporting a support 3. Support 3 supports a C-arm 4 which is fixed to the horizontal section of support 3. The C-arm 4 in its turn supports at one of its ends an X-ray source 5, and at the opposed end an X-ray sensor 6. The support 3 is vertically movable in order to adjust it to the different heights of individual patients. Moreover, a device 7 for the positioning of the patient is present, which is also fixed to support 3.

The floor-fixed, wall-mounted set-up apparatus 21 is supported by a base 10 of limited dimensions, which unloads on the ground a portion of the weight of the apparatus 21, and by a bracket 12 which fixes it to the wall 8.

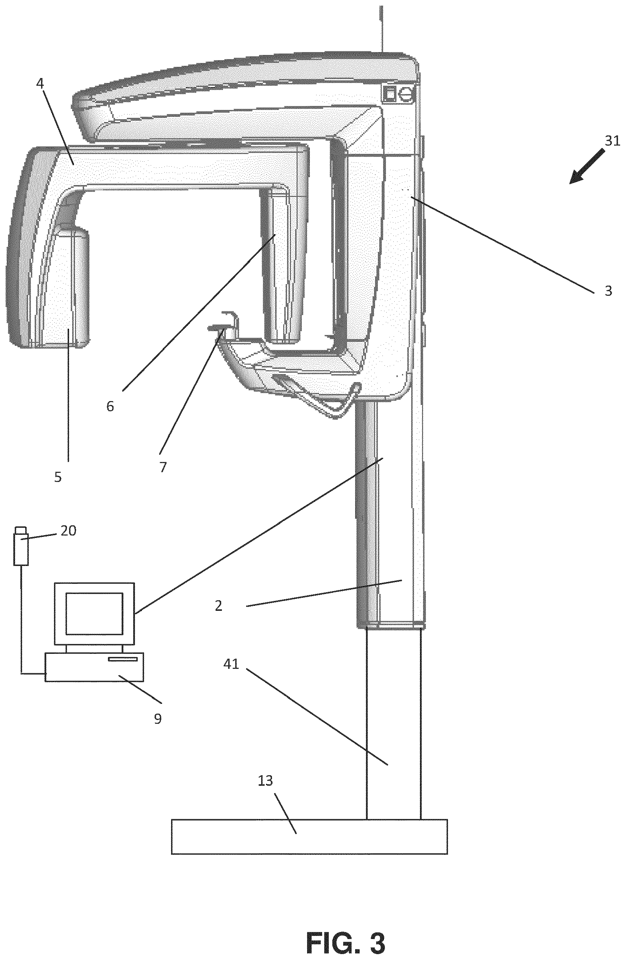

In Figure 3, 31 indicates a stand-alone apparatus capable of performing panoramic radiographies. The apparatus 31 comprises a post 2 supporting a support 3. Support 3 supports a C-arm 4 which is fixed to the horizontal section of support 3. The C-arm 4 in its turn supports at one of its ends a X-ray source 5, and at the opposed end a X-ray sensor 6. The support 3 is vertically movable in order to adjust it to the different heights of individual patients. Moreover, a device 7 for the positioning of the patient is present, which is also fixed to support 3.

The weight of the stand-alone apparatus 31 is totally supported by the post 2 and a base 13; generally, the bracket 12 is not needed.

The visualization of the images acquired by the apparatuses 11, 21, 31 occurs on an external Personal Computer 9 (PC) linked to the apparatus 11, 21, 31. The link apparatus 11, 21, 31 -PC 9 may occur through a cable or wirelessly. Moreover, the PC 9 allows the control of the apparatus 11, 21, 31, in that it contains the software and the needed electronic components, which are not inside the apparatuses 11, 21, 31. This allows to spare space and weight inside the apparatuses 11, 21, 31.

One of the advantages of the present invention consists in its modularity: as a matter of fact, with a limited number of components the final user can be provided with a complete range of models, making the industrial production particularly advantageous:
- wall apparatus (Figure 1)
   The wall configuration is the least cumbersome and the ergonomically most advantageous in that the feet of patient and operator are completely free. However, it requires the presence of a wall capable of supporting the weight of the apparatus (about 80 kg);
- floor-fixed, wall-mounted set-up apparatus 21 (Figure 2)
   It is an intermediate configuration, which can be useful in case of a wall not capable of supporting all the weight of the apparatus. In this configuration the weight of the apparatus is unloaded to the ground by the post, while the bracket allows to use a smaller base than in the stand alone configuration;
- stand-alone apparatus 31 (Figure 3)
   It is the traditional configuration of the known art (e.g. Morita patent US4365340), necessary when the wall cannot support the weight of the apparatus.

The apparatus 11, 21, 31 according to the present invention can have three different configurations:
1. Apparatus capable of acquiring panoramic radiographies only;
2. Apparatus capable of acquiring panoramic and CBCT volumetric radiographies: in this case two distinct X-ray sensors are needed, or alternatively just one bigger sensor capable of performing both acquisitions;
3. Apparatus capable of acquiring panoramic, CBCT volumetric and teleradiographic images: in this case, a CEPH arm is needed. Such arm carries a device for patient positioning and an X-ray sensor suitable for performing a teleradiography. The teleradiography arm can be:
   - fixed to the apparatus 11, 21, 31 itself (not shown). The synchronization between the X-ray source and the teleradiography sensor occurs through a direct cable linking the two units; or
   - a CEPH arm independent from the apparatus according to the present invention and fixed to the wall (not shown). The synchronization between X-ray source and CEPH sensor can occur through a cable passing inside the wall 8, or wirelessly using optical means (e.g. laser, cameras, etc.).

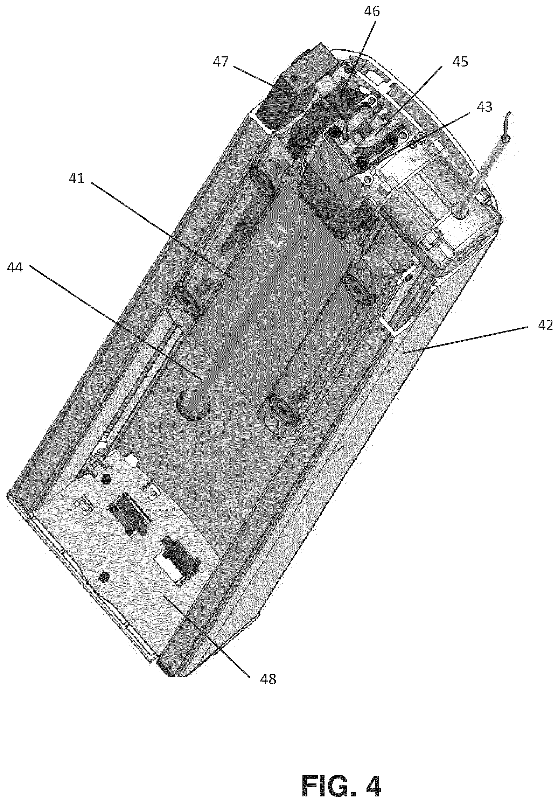

The modularity of the apparatus 11, 21, 31 is made possible by the peculiar configuration of the post 2, shown in detail in Figure 4. The post 2 comprises an internal post 41 and an external post 42. The external post slides along the internal post through interposed sliding and centering longitudinal guides. The external post has an open section on its posterior side, i.e. the side towards the fixing wall, at least for part of its axial extension, so as to allow free vertical sliding notwithstanding the presence of a back bracket for fixing the internal post 41 to the wall. The post has preferably a polygonal transversal section and is made of a tubular bar.

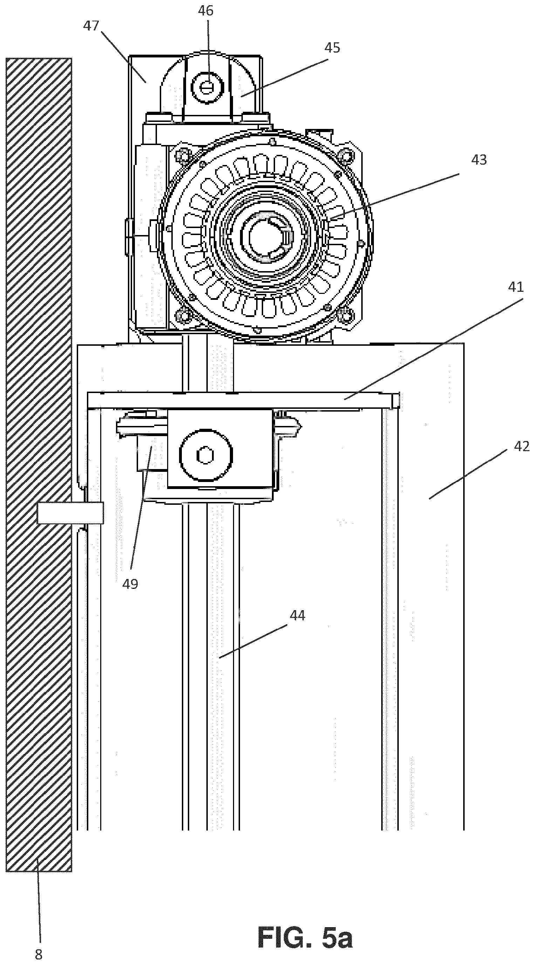

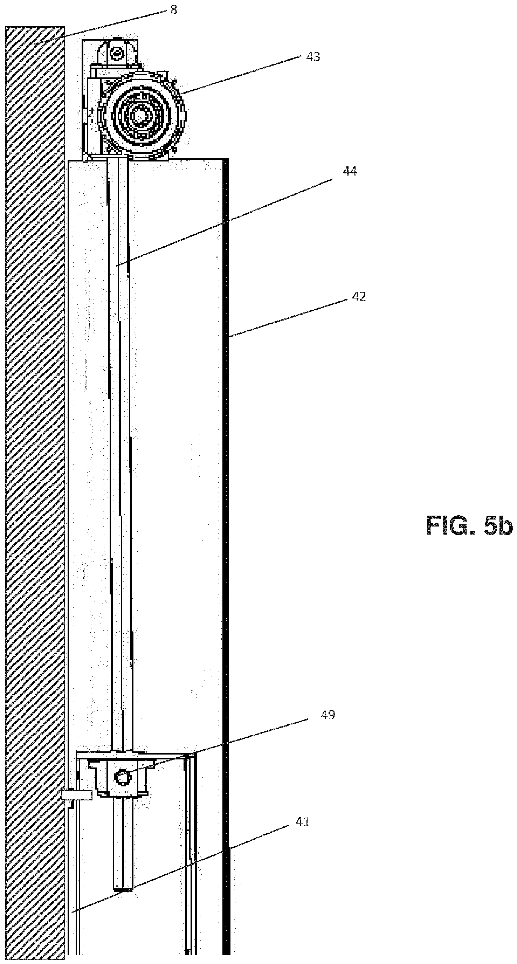

The vertical movement of the device is allowed by the sliding of the mobile external post 42 with respect to the fixed internal post 41, which is fixed to the wall or to the floor or to both, through a telescopic movement. The mobile external post 42 supports a motor 43, moving an endless screw 44. On motor 43 there is a hooking 45 on which a pin 46 is present. At the other end of pin 46 there is a block 47, in its turn fixed to external post 42. The support 3 is fixed to the external post 42. The endless screw 44 is advantageously housed inside the tubular internal post 41, a lead screw nut 49 being supported by the head of said internal post 41, while the endless screw 44 is rotatably operated around its axis by the motor, which in its turn is fixed in a fixed way to the top head of the external column. This configuration entails important advantages, which are clarified with reference to Figures 5a and 5b in the description that follows.

In Figure 5a a vertical section of the post 2 is shown, with the support 3 in the lowest possible position (i.e. the support 3 is at its minimum distance from the floor). Figure 5a shows that the lead screw nut 49 is integral to the internal post 41: when the motor 43 is operated, the lead screw nut 44, screwing and unscrewing on the lead screw nut 49 which remains fixed, lifts or lowers the post 42 and therefore the support 3, allowing to position the apparatus 11, 21, 31 at the height suitable for the individual patient.

The functioning of the motor 43 is inverted with respect to the normal functioning of a lead screw nut motor, in that normally the motor and the endless screw are fixed and the lead screw nut slides on the endless screw, as occurs e.g. in the apparatus described in Morita patent US4002915.

In particular, with respect to the apparatus described in Morita patent US4002915, the advantage of the present invention consists in the fact that here the fixed part remains hidden to the user. In the case of Morita, instead, the fixed top part of the motor 13 must be hidden from view through the use of dedicated housings 21 or open guides 231 (these numbers refer to Morita patent US4002915). This design choice makes the apparatus according to the present invention made of a lower number of pieces, and therefore simpler and cheaper to produce, and moreover it reduces the weight that must be supported by the wall.

In Morita patent US4002915 the endless screw 17 must have two fixing points 124 to the wall (these numbers refer to Morita patent US4002915). In the present invention, instead, the endless screw 44 has one fixing point to the wall only, which is the fixing point of the lead screw nut 49: the advantage lies in the fact that the stroke of the post is linked to the length of the endless screw 44 only, which is not fixed to two fixing points, a higher and a lower one. This allows to choose the length of the internal post on the basis of structural considerations concerning the wall and the apparatus only, and not the lower fixing point or to the stroke of the apparatus.

This concept is better shown in Figure 5b, where the vertical section of post 2 with support 3 (not shown) is in the highest allowed position (the support 3 is at its maximum distance from the floor). In this case the external post 42 is totally lifted, and it is apparent that the only fixing point to wall 8 is the lead screw nut 49 fixed to the internal post 41. The lower end of lead screw nut 44 is free. This kind of structure allows the maximal freedom in the choice of the internal post 41 length, which can be minimal (about 40-60 cm), and therefore completely hidden to the view for the configuration of the wall apparatus 11, or of the necessary length (about 180 cm) for the floor-fixed, wall-mounted set-up apparatus 21 and the stand-alone apparatus 31.

The internal post 41 is the element allowing the modularity of the configuration of the apparatuses 11, 21, 31: as a matter of fact, using a post 41 of suitable length, it is possible to obtain the wall apparatus 11 of Figure 1 (short post) or the floor-fixed, wall-mounted set-up apparatus 21 of Figure 2 or the stand alone apparatus 31 of Figure 3 (long post). This is particularly advantageous from the point of view of the industrial production, in that the bar is the same, both for the short post and the long post; said bar must only be cut at the desired length, while all the rest of the apparatus remains unchanged.

Moreover, in the case of the wall apparatus 11, this allows to use a metallic counter-plate of reduced dimensions, and therefore has a lower impact on the masonry work which are needed in order to install the apparatus 11 in the dental practice.

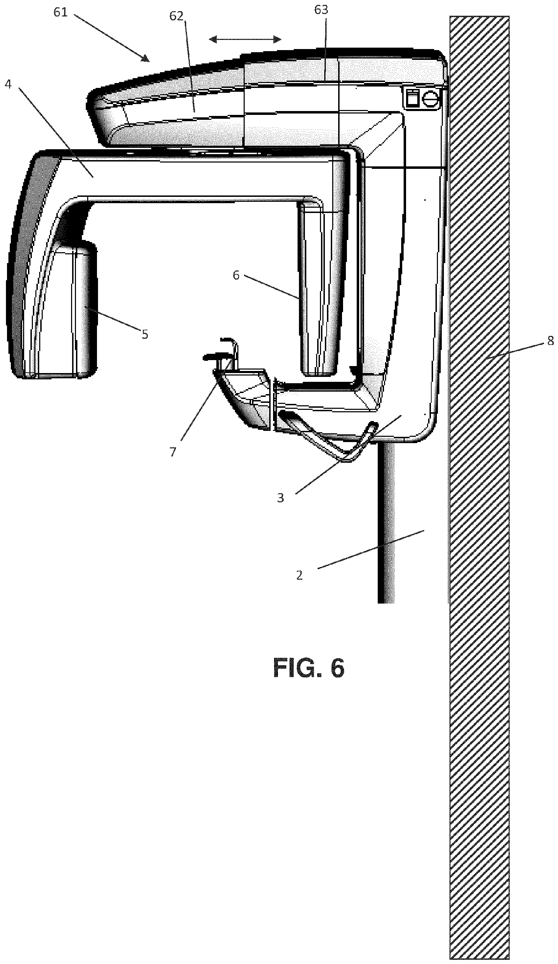

In Figure 6, a different embodiment 61 is shown, wherein the horizontal section of the support 3 supporting the C-arm 4 is made not of only one portion, but of two portions: a mobile portion 62 sliding through a telescopic device on a fixed portion 63, which is integral to the support 3.

With this configuration the C-arm 4 shifts in Y direction, allowing therefore not only an increased freedom of acquisition trajectories, but above all the possibility to make the apparatus according to the present invention particularly compact in the resting position (i.e. when not in use).

In other words, the apparatus has two positions:
- A resting position, wherein the apparatus is not in use and its telescopic arm is retracted towards the wall; therefore, the apparatus shows minimal encumbrance;
- A working position, wherein the apparatus is ready for acquiring images and its telescopic arm is extended in a direction opposite to the wall; therefore, the apparatus shows a bigger encumbrance.

The movement of retraction and extension is obtained through the already present motor moving the C-arm in the Y direction. Nonetheless, the apparatus can acquire radiographic images only when it is in its working position, i.e. when starting a new acquisition, the apparatus has first to pass from its resting position to its working position, and second the apparatus can move along the customary trajectories to perform an acquisition through the customary motors and movements. This may require providing the apparatus with an extra stroke with respect to the Y movement it normally needs to perform acquisition trajectories.

In particular, there is provided a simpler configuration (shown in Figure 6) wherein the patient positioning device 7 is traditionally fixed to the support 3.

In an alternative embodiment, there is provided the fixing of the X-ray source 5 and sensor 6 to a modified arm, which is fixed to the top part of the apparatus (11, 21, 31).

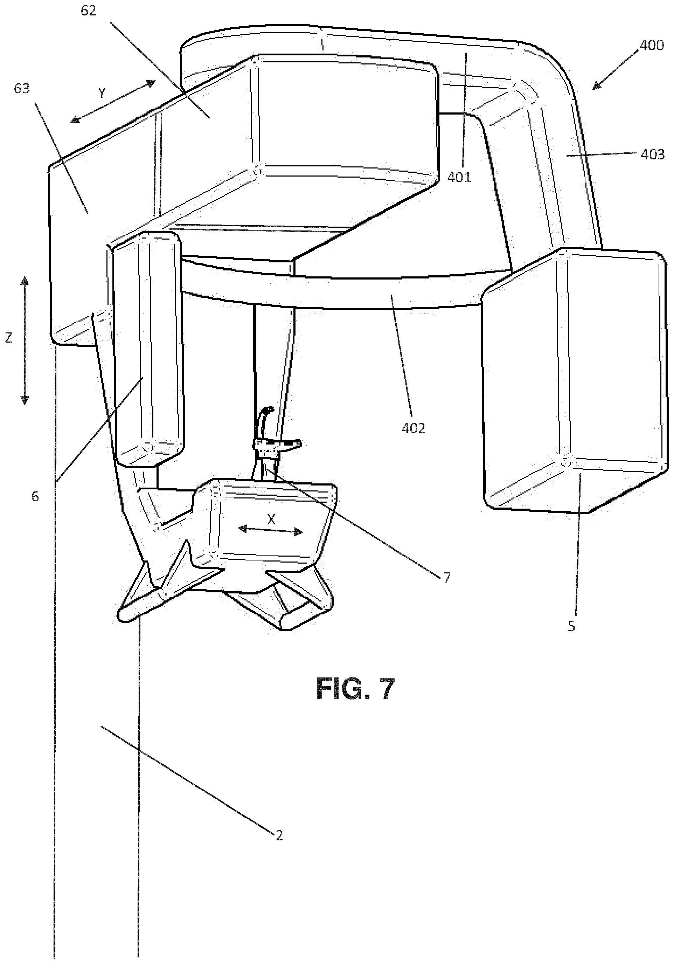

In Figure 7 the most advantageous embodiment is shown, wherein the support of X-ray source 5 and sensor 6 is fixed to a telescopic structure consisting of a strut subdivided into a fixed portion 63 fixed to the wall and a mobile portion 62 linked to the first. The patient positioning device is fixed to the fixed portion 63, which is linked to the portion 62 supporting a C-arm modified, so that the C-arm itself can be installed from above with a marked simplification of the mechanical structure. In particular, all that is fixed to the mobile portion 62 (X-ray source 5, sensor 6) can be moved along the Y axis so that the apparatus 11, 21, 31 is less cumbersome while not in use. Moreover, in this embodiment it is possible to hold the top head of the patient (e.g. through temple-holders and front-holders), fixing it directly to the fixed portion 63 with a marked mechanical simplification.

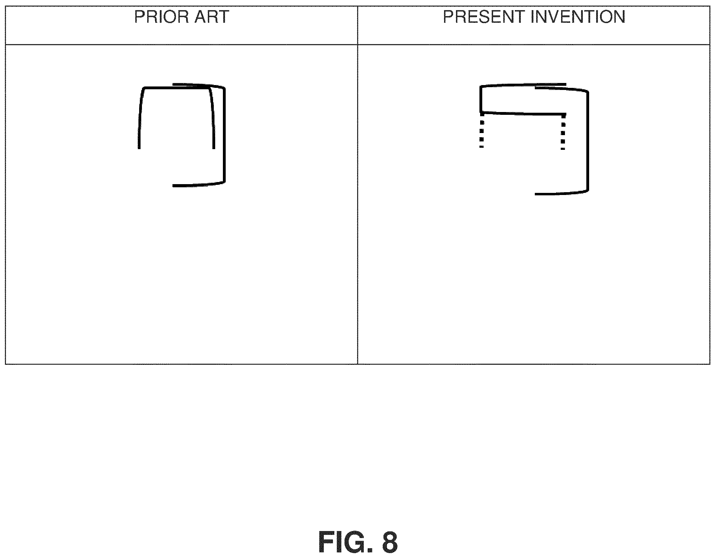

According to a particular embodiment, schematized in Figure 8, the assembly of the stationary arm, patient support and X-rays group is made so as to have the shape of a double opposed C, with the two Cs articulated to each other and angularly displaceable to each other.

In such an embodiment, the X-ray source 5 and sensor 6 are supported by an arm having a C or set down U shape indicated with 400. The top arm 401 is supported by the mobile terminal portion 62 and is not hanging from it, but it is rotatably supported around a vertical axis from the top side of the mobile portion 62, ensuring a simpler mounting of the arm itself with respect to the version wherein said arm is hanging.

The second lower arm 402, parallel to said first arm 401 articulating with the mobile portion 62, supports on its free end the sensor 6. On the other hand, the source 5 is fixed at the lower end of the third transversal arm 403, linking said two horizontal or substantially horizontal 401 and 402; the position of the free end of the arm 402 to which the sensor 6 is fixed with respect to the end of the intermediate arm 403 supporting the source 5 is such that source and sensor are diametrically opposed to each other with respect to the patient, as it is necessary for acquiring patient images.

According to a further embodiment the arm 401 and/or arm 402 can be telescopically extendible, manually o through suitable motorizations, so as to approach and distance source 5 and sensor 6 to each other, therefore modifying acquisition parameters and in particular the magnification or even bringing the sensor 6 in the position for acquiring CEPH images.

Analogously, in alternative or in combination with the features described in the preceding paragraph, the vertical arm 403 can be extendible so as to vertically move the source and the sensor so as to acquire different areas of the patient.

In the version according to Figure 7, the support group 3 and the X-ray group 400, 5, 6 have each a C configuration respectively, wherein the two C are opposed to each other with their top arm or ends overlapping to each other. This is a marked structural alternative with respect to the traditional structure shown in Figures 1-3, which simplifies and makes the mounting operations easier and safer. The difference between the two structures can be better appreciated observing Figure 8.

According to a further feature, in particular the sensor 6 can be fixed or detachable thanks to detachable fixing means on the support arm 4, 402, in any of the described and illustrated versions.

In this case, e.g. the telescopically extendable version of at least the lower arm 402 supporting said sensor 6 can allow to adjust the enlargement ratios in an optimized way for each different kind of sensor 6 mounted on said arm 400. This has advantages especially for the version of the apparatus capable of acquiring the three different kind of radiographic images described above.

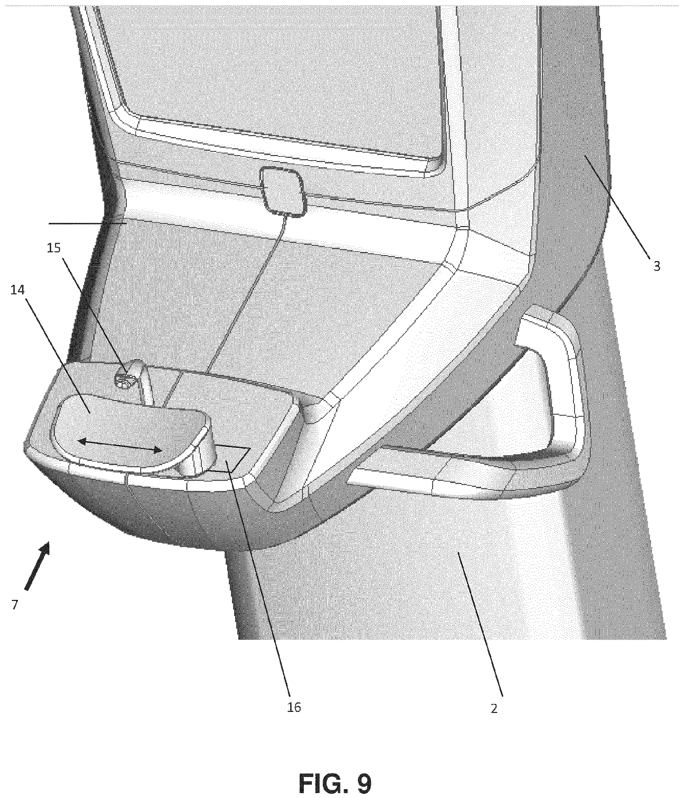

In a preferred embodiment shown in Figure 9 (having the lowest cost, minimal dimensions, lowest weight), the motor for the movement of the C-arm in the X direction is absent. As a consequence in order to acquire the side portions of the patient (e.g. mandibular condyles or molar teeth) it is necessary to position the patient so that her/his anatomic portion to be acquired is as near as possible to the centre of rotation.

As a consequence, the patient positioning device 7 can be positioned in one of at least three distinct positions: a central position for panoramic acquisitions and in general most kind of acquisitions, and two lateral positions (right and left) which are needed to acquire the respective lateral portions of the patient.

In Figure 9, the patient positioning device is made of a chin rest 14 and a bite 15, integral to each other, which can slide inside a slot 16 in the support 3; moving sideways the chin rest 14 all the patient positioning device 7 is moved.

The chin rest 14 can have a continuous movement with respect to the central position, with a block device once the desired position is reached, or can have snap-fixed positions (at least three: left, central, right). The snap movement has the advantage of being more stable and to require a lower number of components, which makes it cheaper to produce.

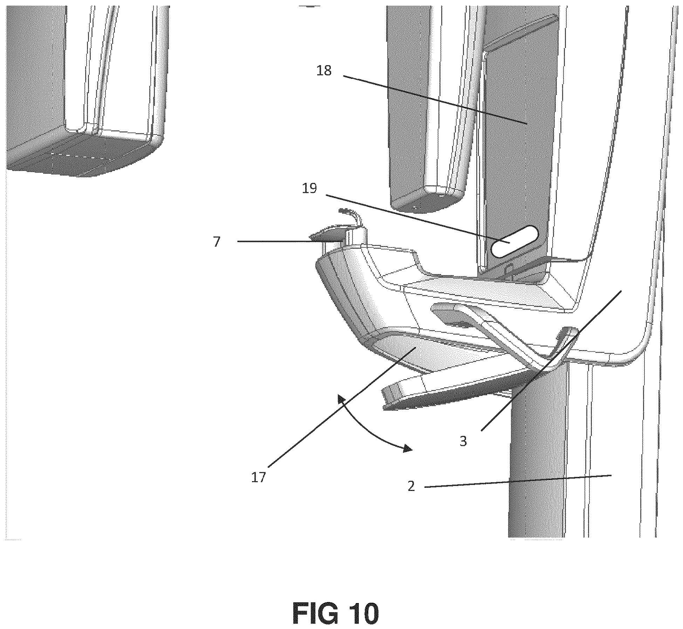

The patient positioning device 7 is supported on a supporting arm integral to the support 3. The patient positioning device 7 is moved in a totally manual way: the absence of motors and the choice to use the snap device frees up space which allows to obtain a small storage compartment 17, shown in Figure 10, which can be used to store the accessories needed for patient positioning: e.g. disposable protections covering the bite, and other accessories of the device itself like temple-holders, bite for edentulous patients, etc.

In a preferred embodiment, the storage compartment 17 can be provided with a source of UV light (not shown), allowing to disinfect the accessories while they are inside the storage compartment.

Moreover, the patient positioning device has a mirror 18 allowing the patient to look herself/himself in the mirror, but above all allowing the professional operator to see patient's face while the operator stands behind her/him, and to check her/his correct centring with respect to the patient positioning device 7 itself. The mirror 18 has at least a backlit slot 19, which allows to detect in a simple way when the apparatus is electrically fed. In the mirror further backlit slots can be present, which can work as references for the positioning of the patient. This design choice was made in that the positioning of the patient in front of the post allows to further reduce the dimensions of the apparatus.

In the wall configuration, the post allows the insertion of an anatomic portion of the patient (hand, knee) between the lower basis of the post (Figure 4) and the floor, and this could lead in the worst case to patient lesions. To prevent this risk, a crushing-preventing device was inserted, blocking the sliding of the external post 42 when the post 42 itself hits against a body which causes its slight lifting.

The lower base 48 has a backlash with respect to post 42; in normal conditions the force of gravity keeps it in its lowest position. When the base 48 moves from its lower position, the sliding of the external post 42 on internal post 41 is blocked.

In order to ease ergonomics, ease of construction and substitution of the shooting button, the shooting button 20, controlling the start of the X-ray generation by the X-ray source 5, can be linked not directly to the apparatus according to the present invention, but to an external Personal Computer (PC) 9, controlling the functioning of the apparatus and allowing to visualize images. Linking the shooting button 20 to PC 9 allows to use an USB port, which is a common, versatile interface offering good guarantee of functioning.
11 Wall apparatus
21 floor-fixed, wall-mounted set-up apparatus
31 stand alone apparatus
61 telescopic strut apparatus
2. post
3. support
4. C-arm
5. X-ray source
6. X-ray sensor
7. patient positioning device
8. wall
9. Personal Computer
10. base
12 bracket
13 base
14 chin rest
15 bite
16 slot
17 storage compartment
18 mirror
19 backlit slot
20 shooting button
41 internal post
42 external post
43 motor
44 endless screw
45 hooking
46 pin
47 block
48 lower base
49 lead screw nut
62 mobile portion
63 fixed portion
400 C or set down U arm
401 top arm
402 lower arm
403 intermediate arm.

## Claims

1. Apparatus (11, 21, 31) for acquiring panoramic and optionally CBCT volumetric images, comprising a X-ray source (5) projecting a collimated X-ray bundle through a patient, a bi-dimensional X-ray sensor (6) placed so as to measure the intensity of the radiation after it crossed the patient, a support group supporting said source (5) and sensor (6), which group comprises a C-arm (4) to which said sensor and said source are fixed/fixable, said C arm (4) being fixed to a vertically mobile support (3) supported by a post (2); a mechanical system capable to allow the rotation and the translation of said C-arm around the patient, so as to acquire radiographic images from different positions; a patient positioning device (7) for positioning the patient in an intermediate position between said X-ray source (5) and said sensor (6);
**characterized in that**
- the said mobile support (3) having a C shape and wherein said mobile support (3) and said support group (400 5, 6) have a double C configuration and wherein the C arm of said mobile support (3) and of the said support group (400, 5, 6) are opposed to each other with their top arms or ends overlapping to each other,
**wherein**
- said C or set down C shaped arm of the said mobile support (400) comprises a second lower arm (402), parallel to a first arm (401) articulated to a mobile portion (62), supporting at its free end said X-ray sensor (6), said X-ray source (5) being fixed at the lower end of a third transversal arm (403) linking said horizontal or substantially horizontal arms (401) and (402), while the position of the free end of arm (402) to which sensor (6) is fixed is such that sensor and source are diametrically opposed to each other with respect to the position of the patient as requested for acquiring radiographic images.

2. Apparatus (11, 21, 31) for acquiring radiographic images according to claim 1, wherein the vertically mobile support (3) comprises a cantilevered branch by which the support group (400) for the X-ray source (5) and the X-ray sensor is supported and which cantilevered branch comprises two portions: a mobile portion (62), supporting the group supporting said source (5) and sensor (6), which mobile portion (62) slides through a telescopic device in the direction of the Y axis with respect to a fixed portion (63), which is integral to support (3); the sliding of said mobile portion (62) relatively to the said fixed position (63) defining a resting position with a minimal dimension, the mobile portion (62) being in the maximal re-entry condition with respect to the fixed portion (63) in said resting position.

3. Apparatus (11, 21, 31) for acquiring radiographic images according to claim 1 or 2, wherein said support (3) supports a C-arm (400) modified so that functional parts, i.e. source (5), sensor (6) and device for fixing patient's front and temples are installed from above.

4. Apparatus (11, 21, 31) for acquiring radiographic images according to claim 1 - 3, having three possible configurations:
- wall apparatus (11), optionally comprising a counter-plate to be bricked in the supporting wall;
- floor-fixed, wall-mounted set-up apparatus (21) comprising a ground base (10) having reduced dimensions, while the post has to be fixed to the wall;
- stand alone apparatus (31) comprising a ground base (13);
wherein the fixed internal post (41) remains unchanged, only its length varies.

5. Apparatus (11, 21, 31) for acquiring radiographic images according to any of the preceding claims, further comprising a CEPH arm (not shown) for acquiring teleradiographies; said CEPH arm being fixed to the apparatus (11, 21,31) itself, or being independent from the apparatus and separately fixed to the wall.

6. Apparatus (11, 21, 31) for acquiring radiographic images according to claim 1, wherein the telescopic movement is present in an independent way on one or more of said three arms:
- arm (401) articulated to the support portion, fixed to the top portion of said C;
- lower arm (402) supporting said X-ray sensor (6);
- transversal arm (403) linking said two horizontal arms (401 and 402).

7. Apparatus (11, 21, 31) for acquiring radiographic images according to any of the preceding claims, wherein the patient positioning device (7) comprises a chin rest (14) and a bite (15), integral to each other, mobile in a continuous or snap way within a slot (16) in support (3); the presence of said slot allowing to obtain at least three distinct positions (central, right, left) for the acquisition of patient's side anatomic portions (mandibular condyles, molar teeth).

8. Apparatus (11, 21, 31) for acquiring radiographic images according to any of the preceding claims, further comprising an external PC (9) for controlling the apparatus and visualizing the acquired images.

9. Apparatus (11, 21, 31) for acquiring radiographic images according to claim 8, further comprising a shooting button (20) controlling the start of X-ray generation by X-ray source (5) linked to said external PC (9) through an USB port.

10. Apparatus (11, 21, 31) for acquiring radiographic images according to any of the preceding claims, wherein said patient positioning device comprises a mirror (18) allowing the patient to look herself/himself in the mirror.

11. Apparatus (11, 21, 31) for acquiring radiographic images according to claim 10, wherein said mirror (18) is provided with a backlit slot (19), allowing to detect when the apparatus is electrically fed.

12. Apparatus (11, 21, 31) for acquiring radiographic images according to claim 10 or 11, wherein the mirror (18) is further provided with backlit slots, working as references for the positioning of the patient.

13. Apparatus (11, 21, 31) for acquiring radiographic images according to any of the preceding claims, wherein said patient positioning device is provided with a small storage compartment (17), useful for storing accessories needed for patient positioning: e.g. disposable protections covering the bite, and other accessories of the device itself like temple-holders, bite for edentulous patients, etc.

14. Apparatus (11, 21, 31) for acquiring radiographic images according to claim 13, wherein said storage compartment (17) is be provided with a source of UV light, allowing to disinfect the accessories while they are inside the storage compartment.
